Europäisches Patentamt

European Patent Office

Office européen des brevets

(11) Publication number: **0 290 395 B1**

## EUROPEAN PATENT SPECIFICATION

(45) Date of publication of patent specification: **26.01.94**

(51) Int. Cl.5: **C12N 15/00**, C12N 15/82, A01H 1/00

(21) Application number: **88810280.3**

(22) Date of filing: **03.05.88**

(54) **Plant tissue transformation.**

(30) Priority: **05.05.87 US 27712**

(43) Date of publication of application:
**09.11.88 Bulletin 88/45**

(45) Publication of the grant of the patent:
**26.01.94 Bulletin 94/04**

(84) Designated Contracting States:
**AT BE CH DE ES FR GB GR IT LI NL SE**

(56) References cited:
**EP-A- 0 142 924**       **EP-A- 0 257 472**
**EP-A- 0 270 356**       **EP-A- 0 289 479**
**WO-A-83/01176**         **WO-A-85/01856**
**CA-A- 1 208 146**

**THE EMBO JOURNAL, vol. 3, no. 12, 1984, pages 2717-2722, IRL Press Ltd, Oxford,GB; J. PASZKOWSKI et al.: "Direct gene transfer to plants"**

**J. CELL BIOCHEMISTRY, suppl. 11B, 1987, page 26, abstract F202; S.L. GOLDMAN etal.: "Transformation of Zea mays by Agrobacterium tumpfaciens: Evidence forstable genetic alterations"**

(73) Proprietor: **SANDOZ AG**
**Lichtstrasse 35**
**CH-4002 Basel(CH)**

(84) Designated Contracting States:
**BE CH ES FR GB GR IT LI NL SE**

(73) Proprietor: **SANDOZ-PATENT-GMBH**
**Humboldtstrasse 3**
**D-79539 Lörrach(DE)**

(84) Designated Contracting States:
**DE**

(73) Proprietor: **SANDOZ-ERFINDUNGEN Verwaltungsgesellschaft m.b.H.**
**Brunner Strasse 59**
**A-1230 Wien(AT)**

(84) Designated Contracting States:
**AT**

(72) Inventor: **Alfinito, Sharon Clarice Harvey**
**35718 Chaplin Drive**
**Fremont CA 94536(US)**
Inventor: **Dietrich, Paul Shartzer**
**3949 Bibbits Drive**
**Palo Alto, CA 94303(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

BIOLOGICAL ABSTRACTS, vol. 73, no. 10, 1982, abstract no. 72908, Philadelphia,PA, US; M. OCHO et al.: "Microinjection of nucleic acids into culturedmammalian cells by electrophoresis", & ACTA MED. OKAYAMA 35(5):381-384. 1981

CHEMICAL ABSTRACTS, vol. 103, 1985, page 361, abstract no. 156736a, Columbus,Ohio, US; D.A. SPANDIDOS et al.: "Transformation of mammalian cell byiontophoretic pricking or iontophoretic microinjection",& EUR. J. CELL BIOL. 1985, 37, 234-9

GENE, vol. 41, no. 1, 1986, pages 121-124, Elsevier Science Publishers B.V.(Biomedical Division), Amsterdam, NL; H. MORIKAWA et al.: "Gene transfer intointact plant cells by electroinjection through cell walls and membranes"

Nature 325, 274-276, 1987

Inventor: **Murry, Lynn Ellen**
**159 Los Trancos Circle**
**Portola Valley, CA 94025(US)**
Inventor: **Sinibaldi, Ralph Michael**
**38658 Greenwich Circle**
**Fremont, CA 94536(US)**

**Description**

The present invention relates to a process for introducing DNA into monocotylodenous (monocot) and dicotyledonous (dicot) plant cells utilising a DNA transporting electric current. The process provides for the direct transfer of plasmid DNA into intact monocotyledonous and dicotyledonous tissue.

BACKGROUND OF THE INVENTION

The genetic transformation of plants has been approached thus far in two different ways. Both of these, however, have limitations when applied to monocotyledonous plants, and more particularly to commercially valuable crops, such as the cereals.

The first of these methods relies upon the ability of a region of the tumour-inducing (Ti) plasmid of the soil microbes of the Agrobacterium genus to fuse with a host cell genome upon infection of a host with the bacterium. Genetic manipulation of the plasmid thus allows introduction of foreign DNA into a host cell. However, since monocotyledenous plants are generally not susceptible to this micro-organism, the method has been limited to the transformation of dicotyledonous plants. Though the genetic transformation of Asparagus using this method has been disclosed [WO 86/03776] and Grimsley et al, Nature 325 (1987) 177, have reported the transfer of DNA into cereals via Agrobacterium, it has not yet been demonstrated that actual transformation ie uptake and integration of the exogenous DNA with the host cell genome or expression of the desired new traits, may occur.

The second method involves the direct uptake of nucleic acids by plant protoplasts. Such uptake may be achieved either chemically, ie polyethyleneglycol stimulated uptake as described by Paszkowski et al, Meth Enzym 118 (1986) 668, or by the use of high voltage electrical pulses of microsecond duration. This latter technique has been described either as electroporation or electroinjection, and is believed to work by punching transient fissures in the plant cell membrane through which externally provided nucleic acids may readily pass. See, eg, Fromm et al, PNAS 82 (1985) 5824, Fromm et al, Nature 319 (1986) 791 and WO 87/06614. However, the success of this method depends upon the ability of a mature fertile plant to be regenerated from a protoplast, a feature which in the case of cereals, has been demonstrated only in rice. Attempts have been made to regenerate maize protoplasts into fertile plants, eg Graves et al, Theor Appl Genet 54 (1979) 209, but these have as yet proved unsuccessful.

Investigations have also been performed, utilising the electroporation method, to transfer naked RNA into cells of dicotyledonous plants. Morikawa et al, Gene 41 (1986) 121, have demonstrated that RNA from the Tobacco Mosaic Virus (TMV) may be taken up by whole mesophyll cells of the dicotyledonous plant Nicotiana. This method, however, performed on individual cells, is stated to have produced cells with a low survival rate and it is not clearly demonstrated that the host cells were transformed rather than infected. Further, even if successful electroporation of individual monocotyledonous cells were to be accomplished in the future, there would still remain the problem of regenerating whole plants, and in particular fertile maize plants, from individual cells.

DEFINITIONS

In describing the present invention, the terminology is intended to be used in accordance with its current accepted meaning in the art. To the extent that accepted meanings do not exist or are ambiguous, the following definitions are to control:

"electrotransformation" is the utilisation of a long-term, continuous direct electric current to induce the uptake of DNA into a host or recipient cell and the subsequent integration of the said genetic material into the host cell genome;

"plant tissue" is a population of cells;

"embryo" is the stage in development in which specific organs or organ systems such as roots and shoots, are not visibly differentiated, but cell compartments which will give rise to organs are present;

"callus" is the cluster of plant cells resulting from the tissue culturing of a single plant cell or tissue;

"CM(30)" is an artificial corn medium, the composition of which is illustrated in Table A;

"MTM" is an artificial liquid medium useful for the culturing of maize tassels, the composition of which is described by Pareddy, Greyson and Walden; Planta 170 (1987) 141;

"BSS" is an artificial liquid medium for Brassica stem strips, the composition of which is illustrated in Table B.

"TMM" is an artificial medium for the culturing of tomato embryos the composition of which is illustrated in Table B.

a "protoplast" is a plant cell from which the cell wall has been removed, usually by enzyme digestion, but which is bounded by a cell membrane;

"meristem" or "meristematic tissue" is comprised of cells which are fully capable of further division, giving rise in turn to embryonic, primary or secondary tissue;

"genome" as used herein refers to all of the genetic material found in a cell, both chromosomal and extra-chromosomal;

a cell "transformed" by DNA is a cell which contains said DNA or a descendant thereof through mitosis or meiosis, which still retains said DNA sequence in its genome.

"membrane-permeating" agents are agents known as such for mammalian cells;

The present invention relates to a method for transforming plant cell material comprising contacting the cell material with a transformation solution comprising DNA and a cell membrane-permeating agent in the presence of an electrical current for a time sufficient to effect transformation.

The method of this invention has numerous advantages. It allows the penetration of numerous layers of cells such that whole tissues rather than single cells may be used. The cells are not injured; they maintain their viability and can be used for the production of mature, fertile plants. The method also allows for the transformation of plants which had previously not been successfully transformed. The invention also relates to a method for producing a transformed fertile plant comprising contacting plants cells with a transformation solution comprising DNA and a cell membrane-permeating agent in the presence of an electric current for a time sufficient to effect transformation and culturing the thus transformed plant cells under culture conditions.

In order to distinguish the transformation process of the invention from so-called electroporation or electroinjection techniques, in which short pulses (in general of the order of some usec to circa 400msec) of electric current are employed, this transformation process is defined as electrotransformation.

Hence, the present invention utilises a more constantly, longer applied electromotive force (tension) which effects a carrying or transporting of the DNA to and through the cell membrane, the permeability of which is sufficiently enhanced by the membrane-permeating agent.

For the electrotransformation of the invention, any non-protoplastic plant material may be utilised. Thus, for example, the present invention envisages the transformation of plant tissue, plant embryo, meristematic tissue such as tassel or ear meristem, axillary buds, stem strips, callus or cell suspensions. When using corn embryonic tissue, it is preferred that the embryos have reached stage 3 of development as defined by Abbe and Stein in Am J Botany 41 (1954) 286-287, ie at a stage between 22 and 28 days after pollination. A corn embryo at stage 3 is typically about 3mm long and he scutellar node tissue is externally recognisable by the two constrictions which set it off. Within the coleorhiza, the primary root has become recognisable and within the coleoptile the first and second leaves have increased considerably in size. This stage is characterised by the third leaf primordium which has recently arisen. The plant material employed is conveniently in excised form.

The process of the invention is conveniently carried out in a horizontal gel electrophoresis system, eg an agarose gel electrophoresis system, into which the plant material to be transformed and the DNA are placed in wells such that the electric current will run from the DNA to the plant material.

When using embyronic tissue, it is preferred that the embryo is positioned in the well such that the side containing the meristematic tissue is facing the wells containing the transformation solution. A solution containing the DNA, (which may, to facilitate selection at a later stage, contain, for example, a DNA sequence coding for resistance to a specific antibiotic) and a membrane-permeating agent are then placed in wells adjacent to those containing the plant cell material. Preferred membrane-permeating agents are polar, such that they are carried, in association with the DNA, to and through the cell membrane when the tension is applied. Suitable examples of membrane-permeating agents include DMSO, lysolecithin and detergents such as sodium dodecyl sulphate and Triton-X, preferably DMSO. The concentrations of such a membrane-permeating agent uses should be sufficient to render the cell membrane temporarily permeable but without destroying the integrity of the intracellular organelle membranes. Such concentrations may vary within wide ranges and will i.a. depend on the plant material involved. Tougher plant cell material, such as dicotyledonous cell material may stand, for example, a concentration of 10% DMSO. In general, good results will be obtained with a concentration of membrane-permeating agent of from about 1% to about 4%, preferably about 2%, by weight of the transformation solution. A tracking dye may optionally be included in the transformation solution to enable the progress of the vectors to be monitored. Examples of suitable tracking dyes would be bromophenol blue, bromocresol green or xylene cyanol. Such dyes and their use for such a purpose are well known in the art. A low voltage electric current is applied such that it runs in a direction from the vector-containing wells toward the wells containing the plant material. Thereafter, the tissues are washed with sterile liquid and placed on solid medium in order for them to recover from any trauma associated with the electrotransformation process. The period of time spent on the solid medium is dependent on the age and size of the tissues treated and younger and/or smaller tissues may require a longer recovery period prior to the selection process. The tissues are normally kept on the medium for 2-3 days, after which the successfully transformed tissues are selected, which process may, if the transformation vector containing a DNA sequence coding for resistance to a specific antibiotic, be by exposure of the tissues to this specific antibiotic. Those tissues which germinate (develop roots and shoots in the case of embryos) or grow and develop chlorophyll on the selection medium have acquired resistance to the antibiotic by uptake and incorporation of the transforming DNA.

The voltage (tension) applied should be sufficient to assist in the transportation of the DNA and not exceed a tension which would substantially damage the cells. The tension to be employed may vary within wide ranges and will depend on various factors such as the type and form of the plant material employed and the time during which the tension is applied. Thus 50% of corn embyros will survive when put under a tension of 200V for 5 minutes in a gel electrophoresis system as disclosed in Example 1. (Corn is about the toughest monocot, and dicots survive, in general, better than monocots.) Voltages up to 110V may be used without seriously damaging the embryro, although germination may be delayed. Voltages above 200V should in general be avoided. On the other hand, transformation may still be obtained with a tension as low as 8V. In general, satisfactory results will be obtained when applying an electric tension from 40V to 140V, eg from 50V to 140V, preferably from about 50V to 110V and most preferably from 52V to 80V. In the gel electrophoresis system employed in the examples the electrodes are 18cm apart. Thus the application of a tension of eg 50V in such a system will result in an electric field strength of 50V/18cm or 2.78V/cm. This gives an idea of the order of magnitude of electric field strength applied. The electric current strength produced in such systems will depend i.a. on the transformation solution (gel etc) and the voltages applied. In the electrophoresis system employed in Example 1, at the voltage of 52V an electric current of circa 25mA is produced.

The period of time of exposure to the electric current is dependent on various factors such as the distance between the wells containing the tissue and the wells containing the transforming solution and also on the size of the tissue sample. Thus the minimum period of time is that necessary for the DNA in the transforming solution to flow, under the influence of the electric field, into the area of the gel containing the plant material samples. For example, if the distance between the two sets of wells is 1 mm at initiation of the current, at a voltage of 52V it will take about 13 to 15 minutes for the transforming solution to move past a corn embryo and it will take about 20 minutes for the solution to move past a larger tissue such as a tassel initial. In general transformation will be obtained in good yields after exposure to the electric tension for 5 to 25 minutes, in particular for 10 to 20 minutes.

The distance between the set of wells, ie those containing the tissue sample and those containing the transforming solution, is dependent on the strength of the transforming solution and the concentration of the support gel such that the distance is preferably higher when the gel is more dilute.

The DNA to be employed is conveniently in vector form, preferably in plasmid form, which plasmid is genetically manipulated using standard recombinant DNA techniques well known to those skilled in the art, so as to contain DNA with which it is desired to transform the plant tissue.

DNA suitable for use in this invention would include any DNA originating from a source other than the host or recipient cell. Examples of valuable such DNA which may be used in the electrotransformation process of this invention could thus include DNA encoding zein, the storage protein of corn, or tissue specific promoters such as those from maize genes, which may be used in chimeric constructions. An example of such a promoter could be the alcohol dehydrogenase (ADH) promotor which is inducible in roots.

A preferred class of DNA for use in this invention can be classified as foreign DNA. The term foreign DNA as used herein refers to any DNA originating from a source other than the host or recipient species. Foreign DNA includes for example, non-host plant DNA, synthetic DNA sequences, sequences produced by recombinant DNA techniques, bacterial, fungal, viral, animal DNA sequences and the like.

Suitable foreign DNA can include non-host plant promoters such as T-DNA promoters from Ti and Ri plasmids, plant virus promoters such as CaMV, TMV, BMV etc, and the like. Valuable foreign DNA can also include foreign structural sequences from the genes for, eg chloramphenicol acetyl transferase (CAT), neomycin phosphotransferase II (npt-II), nopaline synthase (nos), $\beta$-galactosidase ($\beta$-gal), the glyphosate resistance gene (EPSP which is the enzyme conferring resistance to glyphosate-5-enolpyruvylshikimate-3-phosphate synthase) and Bacillus thuringiensis type genes coding for a crystal protein insect toxin. See, eg Adang et al, Gene 36 (1985) 289; Wong et al, Proc 9th Int Spore Cong (Hoch & Setlow Eds, 1985). Examples of Bacillus thuringiensis type genes coding for a crystal protein insect toxin include the B.t. var kurstaki gene coding for a protein toxin toxic to lepidopterous larvae, particularly Noctuidea, more particularly Heliothis zea and Heliothis virescens and Spodoptera species such as Spodoptera exigua and Spodoptera frugiperda, and the B.t. var tenebrionis gene coding for a protein toxin toxic to Coleoptera pests, particularly Chrysomelidae, more particularly Diabrotica species such as D longicornis, D undecimpunctata and D virgifera and Leptinotarsa species such as L decemlineata. The aformentioned Heliothis, Spodoptera, and Diabrotica species are pests infecting crops such as corn. Foreign DNA also includes synthetic genes, such as synthetic DNA sequences based upon native host plant genes, eg a zein gene altered so as to change the amino acid composition of the maize storage protein. The foreign DNA of the present invention preferably comprises chimeric constructions such as heterologous promoter/structural sequence combinations. Examples of such heterologous constructions include the Bt toxin gene under the control of the ADH promoter, and selectable markers such as the CAT or npt-II structural sequence under the control of a T-DNA promotor or a CaMV promoter. Such combinations may be constructed according to standard recombinant techniques such as those illustrated in Maniatis et al, Molecular Cloning: A Laboratory Manual - (1982) and DNA Cloning Vol I & II (D Glober, Ed 1985). Other DNA sequences or combinations of such which may be used in the present invention will be readily apparent to one of ordinary skill in the art.

In a preferred embodiment, the plant material is transformed by a selectable marker gene so that identification of successfully transformed tissues is facilitated. Examples of such marker genes are, for example, genes coding for resistance to antibiotics (such as kanamycin, hygromycin, neomycin and chloramphenicol), genes for herbicide resistance and genes for colour (eg the anthocyanin gene). The presence of a gene coding for an antibiotic will, for example, enable transformed plant cells to survive and grow in a medium containing the selective antibiotic.

In another preferred embodiment, the plant material is transformed by genes coding for traits which have high commercial or agricultural value, such as resistance to insects, resistance to herbicides, resistance to viruses, resistance to fungi, or for enzymes which will interfere in particular biochemical pathways, such as those leading to the synthesis of essential amino acids.

In a particularly preferred embodiment, the plant material is transformed by DNA which contains a selectable marker gene and one or more DNA sequences encoding other desired traits. The use of a marker gene means that successfully transformed material may easily be differentiated from un-transformed material thus facilitating the later screening of the material for that which has incorporated into its genome the co-transferred gene, or genes, which may themselves not be easily selectable.

The plant material transformed according to the method of the invention may be grown and regenerated into fertile plants.

Genetically transformed healthy fertile monocotyledonous plants are novel. The invention accordingly provides monocotyledonous plant material comprising in its genome DNA originating from a source other than the host or recipient species and capable of regenerating into healthy, fertile plants.

The term healthy as used herein is intended to distinguish the plants of the invention from plants which are naturally infected by viruses and the like.

The invention further provides healthy, fertile monocotyledonous plants comprising in their genome DNA originating from a source other than the host or recipient species and parts thereof, in particular seeds of such plants.

Preferred monocotyledonous plant material or plants according to the invention are crops of the Gramineae family, particularly cereals including rice, wheat, the millets, barley, sorghum, rye, oats, triticale and corn, more particularly cereals selected from wheat, the millets, barley, sorghum, rye, oats, triticale and corn and most particularly corn.

It should however be appreciated that the method of the invention is also a convenient alternative method for transformation of dicotyledonous plants including Brassica oleracea species, tomatoes, sunflower, carrots, cucurbits, potatoes, soybean, cotton and the like.

The following examples illustrate the invention but are not intended to limit its scope.

Temperatures are given in degrees centigrade (°C) and percentages (%) are by weight. The electrotransformation is essentially effected at room temperature, whereby, however, the temperature of the electrophoresis system may increase under the influence of the electrical tension.

TABLE A

CM(30) medium (liquid or solid)

MS major salts

| | |
|---|---|
| $NH_4NO_3$ | 1.65 g/l |
| $KNO_3$ | 1.90 g/l |
| $CaCl_2.2H_2O$ | 0.44 g/l |
| $MgSO_4.7H_2O$ | 0.37 g/l |
| $KH_2PO_4$ | 0.17 g/l |

MS minor salts

| | |
|---|---|
| $H_3BO_3$ | 6.20 mg/l |
| $MnSO_4.H_2O$ | 16.80 mg/l |
| $ZnSO_4.7H_2O$ | 10.60 mg/l |
| KI | 0.83 mg/l |
| $Na_2MoO_4.2H_2O$ | 0.25 mg/l |
| $CuSO_4.5H_2O$ | 0.025 mg/l |
| $CoCl_2.6H_2O$ | 0.025 mg/l |

Vitamins

| | |
|---|---|
| Thiamine HCl | 0.25 mg/l |
| L-asparagine | 13.2 mg/l |
| Glycine | 7.7 mg/l |

Carbon Source

| | |
|---|---|
| Sucrose | 20 g/l |
| Agar (for solid) | 8 g/l |

Distilled water to 1 litre

**TABLE B**

BSS MEDIUM

| | |
|---|---|
| 4X Difco salts mixture[1] | 500 ml/l |
| nicotinic acid | 0.5 mg/l |
| pyridoxine HCl | 0.5 mg/l |
| thiamine HCl | 1.0 mg/l |
| inositol | 100 mg/l |
| naphthalene acetic acid | 0.2 mg/l |
| benzyl adenine phosphate | 1.0 mg/l |
| sucrose | 30 g/l |
| agar (for solid) | 16 g/l |
| distilled water to 1 litre | |
| pH adjusted to 5.8 | |

TMM MEDIUM

| | |
|---|---|
| 4X Difco salts mixture[1] | 250 ml |
| nicotinic acid (0.5mg/ml) | 1 ml |
| pyridoxine HCl (0.5mg/ml) | 1 ml |
| thiamine HCl (0.5mg/ml) | 2 ml |
| inositol (100mg/ml) | 1 ml |
| IAA (0.1mg/ml) | 5 ml |
| sucrose | 20 g |
| agar (for solid) | 8 g |
| distilled water to 490ml | |
| pH adjusted to 6.0 | |

[1] Difco salts mixture is a commercially available mixture of the Murashige and Skoogs ("MS") major and minor salts and 4X relates to the strength of the stock solution.

EXAMPLE 1

Into each of eight wells in a 0.7% agarose gel is placed one stage 3 excised corn (P3780) embryo (refrigerator-synchronised for 7 days). In each of eight additional wells, running parallel to and 1 mm from the first eight wells, is placed 15 $\mu$l (10$\mu$g) of plasmid DNA (H83E or H83R), 2 $\mu$l of bromophenol blue dye and 2% of DMSO, the balance being distilled water. The embryo is positioned in the well such that the side

8

of the embryo containing the meristematic tissue is towards the wells containing the transformation solution. The plasmids H83E and H83R each contain the pUC 8 plasmid with a cauliflower mosaic virus (CaMV) 35S promoter [nucleotides 7013 to 7436, see Hohn et al, Curr Top Microbiol Immunol 96 (1982) 193] a hygromycin phosphotransferase (HPT) coding sequence [the Bam H1 fragment from pLG83, see Gritz and Davies, Gene 25 (1983) 179] and a nopaline synthase (NOS) terminator [nucleotides 682 to 437, see Bevan et al, Nucleic Acids Res 11 (1983) 369]. The HPT sequence is in the sense orientation relative to the promoter and terminator sequences in plasmid H83E and in the antisense orientation in plasmid H83R.

Each gel is placed in a horizontal gel electrophoresis system (BRL H6) with 450 ml of sterile tris-acetate-EDTA running buffer (pH 8.0). Gels are exposed to an electric current of 52V, running in the direction from the DNA to the embryos, for a period of either 10, 12.5 or 15 minutes. After exposure, the embryos are rinsed with sterile CM(30) and placed on solid CM(30) medium for 3 days. The embryos are then transferred to CM(30) medium containing 100 $\mu$g/ml of hygromycin. The embryos are scored at 11 and 14 days after electrotransformation treatment, with the following results:

At 11 days all embryos had germinated (had developed roots and shoots). At 11 and 14 days the germlings under Table C were green, indicating acquired resistance to hygromycin by incorporation of the pLG83 gene. Control (a) corresponds to tissues electrotransformed as in the tests, but thereafter grown in the absence of hygromycin; control (b) corresponds to tissues exposed to the electric current but in the absence of transformation solution and thereafter grown in the presence of hygromycin. Non-transformed hygromycin selected controls turn chalk white and cease to grow.

## TABLE C

| Day | Control (a) | (b) | H83E 10 | 12.5 | 15 min | H83R 10 | 12.5 | 15 min |
|-----|-----|-----|-----|------|--------|-----|------|--------|
| 11 | 8 | 0 | 4 | 4 | 4 | 6 | 3 | 3 |
| 14 | 8 | 0 | 4 | 4 | 2 | 6 | 3 | 3 |

## EXAMPLE 2

At 14 days after treatment with the plasmid DNA, the green germlings (seedlings) from each test group in Example 1 are collected and ground up, and the nucleic acid is extracted following the cetyltrimethylammonium bromide (CTAB) procedures described by Rogers et al Plant Mol Biol 5: (1985) 69.

The harvested germling tissue for each group is ground to a fine powder in liquid nitrogen or dry ice and placed in a test tube. The mixture is warmed slowly to 65° and solution of CTAB [2% CTAB (w/v), 100 mM Tris (pH 8.0), 20 mM EDTA (pH 8.0), 1.4 M NaCl and 1% PVP (polyvinylpyrrolidone)] at 1 $\mu$l/mg is added, followed by heating at 65° for 3 mins. An equal volume of sevag (CHCl$_3$:isoamylalcohol at 24:1) is added and mixed. The mixture is centrifuged at 11 kx for 30 sec and the top phase is transferred to a new tube, and CTAB (10% w/v) and 0.7 M NaCl are added. Centrifugation is repeated, and the top phase is again separated and diluted with CTAB and NaCl. One volume of CTAB precipitation buffer [1% CTAB, 50 mM tris (pH 8.0) and 10 mM (pH 8.0) and 1 M NaCl] and is heated to 65° for 10 mins. After complete rehydration, the nucleic acids are reprecipitated with 2 volumes of ethanol and then pelleted by centrifugation for 13 - 15 mins in a cold room. Minigels were run on all samples and showed the presence of DNA.

For dot blot analysis, the extracted DNA is heated to 100°, 20 X SSPE (3.6M NaCl, 200mM NaH$_2$PO$_4$, pH 7.4, 20mM EDTA, pH 7.4) is added and the solution is cooled and transferred to a nitrocellulose membrane. It is hybridised with nick-translated DNA (BRL kit lot #5210, and H83E). See, Rigby et al J Mol Biol 113 (1977) 237. The nitrocellulose is rinsed in 2 X SSPE and 0.1% SDS and then heated at 50° for 1 hour, followed by agitation in 1X SSPE and 0.1%SDS at room temperature for 1 hour, and then exposed to x-ray film. See, Maniatis et al Molecular Cloning: A Laboratory Manual (1983). The results indicate strong hybridisation, and therefore transformation, occurred in 10% of the dot blot treated samples.

EXAMPLE 3

Tassel initials (1.0 - 1.5 cm long) of maize (cvx. Oh 43 and Se60) are dissected aseptically, as described by Pareddy and Greyson. Plant Cell Tissue Organ Cult 5: (1985) 119, and kept on MTM medium until electrotransformation treatment. Following the procedure of Example 1, th tassels and transforming solution comprising either a) 8 $\mu$l (10 $\mu$g) of H83E plasmid, 2% of DMSO and 29 $\mu$l of distilled water, or b) 15 $\mu$l (10 $\mu$g) of H83R plasmid, 2% of DMSO and 22 $\mu$l of distilled water are placed in a 0.7% agarose gel in an electrophoresis system apparatus and exposed to an electric current of 52V for 13 mins. Following exposure, the tassels are removed to sterile CM(30) medium with 50 $\mu$g/ml of gentamycin added and are rinsed gently for a short period. The gentamycin acts as an anti-bacterial. They are then placed in sterile flasks containing 50 ml of MTM media and the flasks are placed on a window sill. Two days after treatment, 5 $\mu$g/ml of hygromycin is added to each flask.

Six days after addition of the hygromycin, 1 out of 3 H83R-treated tassels had died (had stopped growing and bleached white). Ten days after the addition, 2 out of 15 H83E-treated tassels had died. Thirteen days post-addition, 2 more H83E-treated tassels had died. The remaining tassels continued to grow and stay green through 25 days after addition of the hygromycin. Further, one of the tassels treated with H83E produced pollen.

EXAMPLE 4

Following the procedures of Example 1, stem strips (approx 1x3x4mm) taken from between the upper two nodes of the shoot in the bud of Brassica oleracea [cv italica, CrGC-9 (Crucifer Genetics Co-operative9)] are placed in a 0.7% agarose gel in an electrophoresis system apparatus along with transformation solution comprising 15$\mu$l (10$\mu$g) of pZ025 plasmid DNA, 2$\mu$l of bromophenol blue dye and 2% of DMSO, and exposed to an electric current of 52V for about 15 minutes.

Following exposure, the stem strips are removed to sterile BSS medium for a short time, then to selection medium containing 20$\mu$g/ml of hygromycin for 1 week and finally to medium containing 5 $\mu$g/ml of hygromycin for 1 week. Twenty four stem strips produced callus when transferred to BSS medium without hygromycin. After 2 months they generated shoots and were transferred to soil in a mist chamber to generate roots.

Leaves from two hygromycin-selected Brassica were cut into pieces and subjected to secondary selection on BSS medium containing 25$\mu$g/ml hygromycin. These pieces remained green and formed shoots. When they were transferred to BSS with half strength salts and no hygromycin they also rooted. Leaves of the same two primary transformants were also analysed for the presence of the hygromycin gene. Each leaf (approx 0.2g) was frozen in liquid nitrogen and ground to a powder in a mortar. To this, 15ml ice cold sucrose buffer (containing 15% sucrose, 50mM Tris pH 8.0, and 50mM EDTA) and 0.25M NaCl was added to the tissue in the mortar and grinding continued. The slurry was poured into a 5ml Wheaton ground glass homogeniser and ground by hand for 5-10 passes. The slurry was then transferred to a 1.5ml Eppendorf tube and centrifuged for 3 mins at 6500 rpm. The crude nuclear pellet was then resuspended in 0.5ml ice cold sucrose buffer (as above but without NaCl). 1$\mu$l diethylprocarbonate was added and the suspension was vortexed at room temperature. Next, 5$\mu$l of 20% SDS was added and vortexed, then heated at 70°C for 10 minutes. 50$\mu$l of 5M potassium acetate were added and the solution vortexed. The tube was then cooled on ice for 30 minutes.

The potassium-SDS fraction was precipitated and centrifuged for 15 minutes at 4°C. The supernatant was transferred to a clean 1.5ml tube and repeatedly extracted with phenol:chloroform until the colour disappeared and the interface was clean. An ethanol precipitation was performed and the solution centrifuged for 5 mins at RT to recover DNA.

Transformants were analysed for the presence of the introduced hygromycin gene. The total DNA was quantitated and cut using the restriction enzyme TAQ 1. The DNA fragments were separated using horizontal gel electrophoresis (0.7% agarose) at 70V for about 3 hours and transferred to a Biorad Zeta Probe membrane using the alkaline blotting procedure for DNA capillary transfer as specified in the Biorad Zeta Probe Blotting Membranes Instruction Manual. After pre-hybridisation, hybridisation and washing, performed also according to the above mentioned manual, the blotted membranes were ready for autoradiography.

An isolated 1Kb fragment of the hygromycin gene was labeled with $^{32}$P CTP according to the low melt agarose procedure given in the Amersham Multiprime DNA Labeling Systems Manual Pages 1 to 28. Diagnostic 1.4 and 0.7Kb fragments were present in transformed DNAs and absent from control DNA.

EP 0 290 395 B1

Plasmid pZ025 is the same as H83E (from Example 1) except that the HPT coding sequence consists of the nucleotides 197 to 1251, modified by replacement of a guanine at position 206 by an adenine.

EXAMPLE 5

Following the procedures of Example 1, stage 3 excised corn embryos and the transforming solution comprising 15 $\mu$l (10 $\mu$g) of pZ033 plasmid DNA, 2% of DMSO and 22 $\mu$l of distilled water are placed in a 0.7% agarose gel in an electrophoresis system apparatus and exposed to an electric current of 52V for 13 minutes.

Following exposure, the embryos are rinsed with sterile liquid CM(30) medium and placed on solid CM-(30) medium. No attempt was made to select for the plasmid DNA. The resulting seedlings are raised in the normal manner to adult corn plants, which are then cross pollinated to give $F_1$ plants. Seeds from the cobs of the $F_1$ plants are germinated and grown for one week in order to conduct CAT assays to establish those cobs containing transformants. Additional seeds from the resulting potentially positive cobs are planted and the roots from the resulting plants are harvested and tested in the CAT assay. Of the first eight potentially transformed cobs, two showed transformation.

Leaves of CAT positive $F_1$ transformants were analysed for the presence of the introduced CAT gene sequences. Procedures for extracting, cutting, separating, blotting and probing the DNA are consistent with those given in Example 4. A diagnostic 1.2Kb fragment is released whenever the pZ033 (35S-CAT-NOS) plasmid or genomic DNA transformed with the plasmid is cut with the restriction enzymes Hind III and Eco RI and probed with the CAT gene. In this progeny family, the 1.2Kb fragment is always associated with a 1.4Kb fragment. The DNA extracted from the tassel used to produce this $F_1$ family and from all positive individuals contained the 1.2Kb and the 1.4Kb fragments. In contrast, all controls (nuclear DNA from 3780, total DNA from 3780 and $F_1$ hybrid 3780) lack these fragments.

Plasmid pZ033 contains a CaMV 35S promoter (nucleotides 7069 to 7569) in the SacI site of pUC19 (commercially available from Pharmacia and from US Biochemical), the 773 bp chloramphenicol acetyl transferase (CAT) gen from Tu9 [see, Alton and Vapnek, Nature 282 (1979) 864] whose TagI ends are changed to PstI, in the Pst site, and a NOS terminator (nucleotides 682 to 437) between the PstI and the Hind III sites.

The CAT assay is as follows:

CAT ASSAY

1. Extraction of seedling or other tissue
A Seedling or other tissue is ground in 150$\mu$l (or multiple thereof) 0.25M Tris-HC1 pH 7.8.
B Sample is sonicated (3 pulses on #2 setting) on ice.
C Sample is centrifuged for 3 mins at 12Kg, and the supernatant transferred to a clean tube.
D Sample is heated to 65° in a water bath for 12 mins, then cooled to RT.
E Sample is centrifuged for 30 seconds, the supernatant collected and used for the CAT assay.

11

2 Set up one tube for each reaction. Also set up a control for the CAT from <u>E coli</u>. (13 x 100cm tubes)

|  | Negative Control | Potential Transformant | Positive CAT control |
|---|---|---|---|
| Supernatant | 100μl | 100μl | – |
| 10mM Tris pH 7.8 | – | – | 99μl |
| CAT (0.5 unit/μl) | – | – | 1μl |
| $^{14}$C chloramphen. | 3μl | 3μl | 3μl |
| H$_2$0 * | 57μl | 57μl | 57μl |
| 4mM Acetyl CoA | 20μl | 20μl | 20μl |
|  | ----- | ----- | ----- |
| TOTAL | 180μl | 180μl | 180μl |

* incubate for 5 mins at 37°C to bring the reaction mixture to RT

3 Incubate samples at 37°C for 1-2 hours.

4 Stop reaction with 2ml cold water-saturated ethyl acetate. Add 1ml H$_2$0 to make the interface more visible.

5 Cover tube with parafilm and vortex on #1 for approximately 1 min.

6 Spin in IEC for 3-5 mins on #5.

7 Transfer top layer to small tubes (13 x 75).

8 Dry under N$_2$ in hood till dry (approx 45 mins - avoid overdrying).

9 While samples are drying prepare the TLC tank with solvent, 100ml or chloroform:methanol, 95:5. Place a wick in the tank for all sides.

10 Prepare a 20 x 20cm silica 60 gel TLC plate. Mark a line in pencil 1.5 cm from the bottom. Mark sample positions with intersecting lines. Separation is by ascending chromatography.

11 After the samples have dried down, resuspend them in 30μl of ethyl acetate (not water saturated).

12 Spot samples in very small area with a 10μl micropipette. Have a hair dryer blowing over the plate while spotting.

13 Place plate in the tank and let solvent front reach approx 3mm from the top of the plate (40-50 minutes).

14 Let the plate air dry for 15 or 20 minutes in the hood, then place in a cassette with a piece of film (preflashed) at RT overnight.

15 Develop the film the next day.

<u>EXAMPLE 6</u>

Following the procedures of Example 1, tomato embryos (Burpees Super Beefsteak VFN) were placed in the wells of a 0.7% agarose gel in a horizontal electrophoresis system beside the wells containing a transformation solution consisting of 10μg pZO 60 or PZO 67 plasmid DNA, 2% DMSO and bromophenol blue tracking dye. The embryos were exposed to an electric current of 52V for about 15 minutes.

After electrotransformation, the embryos were transferred to TMM medium for 2-3 days recovery. Then, they were placed on selective tomato medium which contained 25μg/ml hygromycin for 10 days. None of the untreated control seedlings survived this selection. Transformants were then rescued to medium containing 0.5mg IAA and rooted before being transplanted into soil.

Leaves of these primary transformants were analysed for the presence of introduced DNA, specifically the hygromycin gene. Total DNA was isolated from the plant tissue, cut with the restriction enzyme TAQ 1, separated, blotted and hybridised as detailed in Example 4. Diagnostic hygromycin fragments (1.4, 0.7 and 0.4kb) were present in the transformed DNAs and absent from the control DNA.

The plasmid pZO 60 is constructed from the pUC 8 plasmid with the 35S promoter, HPT coding sequence, and the nos terminator of pZO25 (see example 4) immediately followed, in tandem, by a second copy of the 35S promoter, the CAT coding sequence of pZO 33 (see Example 5) and a second copy of the nos terminator.

The plasmid pZO 67 is constructed from the pUC 8 plasmid with the 35S promoter of H83E (see example 1), the B-glucuronidase (GUS) coding region on a Pst 1 fragment from RAJ-260 (Jefferson et al, PNAS 83 (1986) 8447) and the nos terminator immediately followed (in tandem) by the 35S promoter, HPT sequence and nos terminator of pZO 25 (see example 4).

EXAMPLE 7

Following the procedure of Example 1, stage 3 corn embryos are placed in the wells of a 0.7% agarose gel in a horizontal electrophoresis system beside the wells containing a transformation solution comprising 10$\mu$g of Bacillus thuringiensis var kurstaki (Btk)/HPT plasmid (pZO 85-BTK/HPT) DNA, 2% DMSO and bromophenol blue tracking dye. The embryos are exposed to a continuous electric current of 52V for about 15 minutes, after which they are transferred to CM(30) medium and selected for acquired hygromycin resistance. Those germlings showing hygromycin resistance are raised to larger seedlings and tested by standard bioassay procedures for pest resistance to Heliothis species such as Heliothis virescens and Spodoptera species. The transformed seedlings show reduced susceptibility to Heliothis and Spodoptera species compared with untreated standards.

The plasmid pZO 85 is constructed as follows. The SalI-EcoRI fragment containing the B-glucuronidase (GUS) coding sequence from pRAJ 275 (commercially available from Clontech Laboratories) was modified to change the EcoRI site at the 3' end to a PstI site. A feature of this GUS gene is that an NcoI site straddles the initiator codon (ATG) of the coding sequence. pZO 85 consists of the 35S promoter and nos sequences as found in pZO 33 (see Example 5) and the Sal-Pst fragment described above (in place of the CAT gene).

pZO 85-BTK-HPT is prepared by substituting the Nco-Pst fragment of pAMVBTS containing a modified Btk sequence [Barton et al, Plant Physiol 85 (1987) 1103] for the NcoI-PstI fragment of pZO 85. This sequence is immediately followed (in tandem) by the 35S promoter and nos terminator of pZO 33 (see Example 5) with the HPT sequence of pZO 25 (see Example 4).

EXAMPLE 8

Following the procedure of Example 1, stage 3 corn embryos are placed in the wells of a 0.7% agarose gel in a horizontal electrophoresis system beside the wells containing a transformation solution comprising 10$\mu$g of Btt/HPT plasmid DNA (pZO 85-Btt-HPT), 2% DMSO and bromophenol blue tracking dye. The embryos are exposed to a continuous electric current of 52V for about 15 minutes, after which they are transferred to CM(30) medium and selected for acquired hygromycin resistance. Those germlings showing resistance are raised to larger seedlings and tested by standard bioassay procedures for resistance to the corn root worm (Diabrotica ssp). Bacillus thuringiensis var tenebrionis (Btt) produces an insecticidal toxin active against the corn root work and other coleopteran insects.

pZO 85-Btt-HPT is prepared by substituting a modified Nco-Pst fragment of Btt coding sequence [Sekar et al, PNAS 84 (1987) 7036] which has been modified at the ATG start codon to contain an NcoI site and at the 3' terminus of the coding region to produce a PstI site, for the NcoIPstI fragment of pZO 85 (see Example 7). This sequence is immediately followed (in tandem) by the 35S promoter and nos terminator of pZO 33 (see Example 5) with the HPT sequence of pZO 25 (see Example 4).

**Claims**

1. A method for transforming plant cell material comprising contacting the cell material with a transformation solution comprising DNA and a membrane-permeating agent in the presence of an electric current for a time sufficient to effect transportation of the DNA into the plant material and transformation.

2. The method of claim 1 wherein the DNA is vector DNA.

EP 0 290 395 B1

3. The method of claim 2 wherein the DNA is plasmid DNA.

4. The method of claims 1 to 3 employing a horizontal gel electrophoresis system into which the plants cells to be transformed and the DNA material are placed in wells such that the electric current will run from the DNA to the plant cells.

5. The method of claim 4 wherein the membrane-permeating agent is a polar membrane-permeating agent selected from dimethyl sulfoxide, lysolecithin, sodium dodecyl sulphate and other detergents.

6. The method of claims 1 to 5 wherein the plant material is plant tissue, plant embryo, meristematic tissue, axillary buds, stem strips or callus.

7. The method of claim 6 wherein the plant material is corn plant material.

8. A method for producing transformed fertile plants which comprises transforming plant cell material according to the method of claims 1 to 7 and culturing the thus transformed plant cells under culture conditions.

**Patentansprüche**

1. Verfahren zur Transformation von pflanzlichem Zellmaterial, gekennzeichnet durch Zusammenbringen des Zellmaterials mit einer Transformationslösung, die DNA und ein membranpermeabilisierendes Mittel enthält, in Gegenwart eines elektrischen Stroms für eine Zeitspanne, die zur Bewirkung des Transports der DNA in das Pflanzenmaterial und eine Transformation ausreicht.

2. Verfahren nach Anspruch 1, worin die DNA Vektor DNA ist.

3. Verfahren nach Anspruch 2, worin die DNA Plasmid DNA ist.

4. Verfahren nach den Ansprüchen 1 bis 3 unter Verwendung eines horizontalen Gelelektrophoresesystems, in das die zu transformierenden Pflanzenzellen und das DNA Material in Probentaschen eingebracht werden können, so daß der elektrische Strom von der DNA zu den Pflanzenzellen läuft.

5. Verfahren nach Anspruch 4, worin das membranpermeabilisierende Mittel ein polares membranpermeabilisierendes Mittel ist, ausgewählt aus Dimethylsulfoxid, Lysolecithin, Natriumdodecylsulfat oder anderen Detergenzien.

6. Verfahren nach den Ansprüchen 1 bis 5, worin das Pflanzenmaterial ein Pflanzengewebe, ein pflanzlicher Embryo, ein Meristemgewebe, Achsknospen, Stammstreifen oder ein Kallus ist.

7. Verfahren nach Anspruch 6, worin das Pflanzenmaterial Pflanzenmaterial vom Mais ist.

8. Verfahren zur Herstellung transformierter fruchtbarer Pflanzen, das gekennzeichnet ist durch Transformieren von pflanzlichem Zellmaterial gemäß dem Verfahren der Ansprüche 1 bis 7 und Kultivieren der so transformierten Pflanzenzellen unter Kulturbedingungen.

**Revendications**

1. Procédé de transformation d'une matière cellulaire végétale, qui comprend la mise en contact de la matière cellulaire avec une solution de transformation comprenant de l'ADN et un agent de perméation membranaire en présence d'un courant électrique, pendant un temps suffisant pour assurer le transport de l'ADN dans la matière végétale et sa transformation.

2. Procédé selon la revendication 1, dans lequel l'ADN est un ADN vecteur.

3. Procédé selon la revendication 2, dans lequel l'ADN est un ADN plasmide.

14

**4.** Procédé selon les revendications 1 à 3, qui utilise un système horizontal d'électrophorèse sur gel dans lequel les cellules végétales à transformer et la matière d'ADN sont placés dans des puits de façon que le courant électrique passe de l'ADN aux cellules végétales.

**5.** Procédé selon la revendication 4, dans lequel l'agent de perméation membranaire est un agent de perméation membranaire polaire choisi parmi le diméthylsulfoxyde, la lysolécithine, le dodécylsulfate de sodium et d'autres détergents.

**6.** Procédé selon les revendications 1 à 5, dans lequel la matière végétale est constituée d'un tissu végétal, d'un embryon végétal, d'un tissu méristématique, de bourgeons axillaires, de bandes de tige ou d'un cal.

**7.** Procédé selon la revendication 6, dans lequel la matière végétale est une matière végétale du maïs.

**8.** Procédé de production de plantes fertiles transformées, qui consiste à transformer une matière cellulaire végétale selon le procédé des revendications 1 à 7 et à cultiver sous des conditions de culture les cellules végétales ainsi transformées.